Europäisches Patentamt

European Patent Office

Office européen des brevets

(19)

(11) Publication number: 0 364 306
A2

## (12) EUROPEAN PATENT APPLICATION

(21) Application number: 89310582.5

(22) Date of filing: 16.10.89

(51) Int. Cl.⁵: C12N 5/00

(30) Priority: 14.10.88 US 257558

(43) Date of publication of application:
18.04.90 Bulletin 90/16

(84) Designated Contracting States:
AT BE CH DE ES FR GB GR IT LI LU NL SE

(71) Applicant: TUTORTEC INC.
630 South EL Camino Road
San Clemente California 92672(US)

(72) Inventor: Chao, Chung Faye 36, Lane 5
Alley 626 Ting-Chow Road
Taiwan, Roc.(TW)

(74) Representative: Sexton, Jane Helen et al
J.A. Kemp & Co. 14 South Square Gray's Inn
London WC1R 5EU(GB)

(54) Process for producing cultured epidermal sheet, product and method of use.

(57) A method of culturing cells to form graftable sheets of epidermis and freezing these epidermal sheets for later use in autografts or allografts.

EP 0 364 306 A2

## PROCESS FOR PRODUCING CULTURED EPIDERMAL SHEET, PRODUCT AND METHOD OF USE

### Field of Invention

This invention is in the field of cell culture. More specifically this invention is a method for culturing cells to form graftable sheets of epidermis and freezing these epidermal sheets for later use.

### Background

Severe skin wounds have been a serious medical problem since time immemorial. Most often these wounds are caused by burns, of the second and third degree. A major problem for these skin wound victims is how to protect the damaged areas and help them heal. These wounds leave the patient in shock, subject to renal failure, and highly susceptible to infection.

Burn victims with severe burns often do not have sufficient skin remaining to heal. The remaining skin can be damaged itself and therefore unable to grow rapidly. In addition the body may not be able to survive long enough without the protection provided by the skin in order for the remaining skin to regenerate. Burn victims are especially susceptible to infection, as the skin normally provides the first layer of defense against invading organisms. This can also be a problem in other types of wounds where skin is destroyed.

Historically, victims of such wounds were wrapped in bandages to help seal the wounds and keep out infective organisms. However, such procedures were never completely successful. In addition, the bandages may have hindered the healing process.

More recently, surgeons have taken pieces of pig skin to graft over such wounds. These patches were somewhat effective, but created rejection problems, because the human body would react to the pig skin as invading foreign material. In patients with sufficient amounts of remaining skin, so called split-thickness grafts could be made from healthy sections of the patients' skin. However, this creates additional wounds to the body. Also in many burn patients there is insufficient remaining healthy skin for such a procedure.

In the last approximately ten years, the culture of human skin to use for grafting has developed. In the procedures known up until this present invention, small pieces of skin were cultured in vitro to expand these pieces to a size usable for grafts.

This procedure has a major complication, however, in that the pieces of skin must come from the patient on whom the grafts are be performed. In other words, only auto-grafts using such tissue can be performed. The reason for this limitation is that the body will mount an immune response to allo-grafts (where the source of the tissue is other than the patient on whom the grafts are performed), and the patient's body will reject the graft.

This is a major limitation on the usefulness of this culturing procedure. In the first place, many victims needing grafts have major losses of skin over their bodies and therefore do not have a large source of tissue to be used as a starting point for the procedure. As a result, the available tissue must be expanded in culture by this procedure, and then broken up again to create more starting material. This increases the time delay, and therefore the risk to the patient, before grafts can be performed.

In addition, because these patients have had major trauma to their bodies they are in urgent need of skin grafts -the sooner the skin is available for grafting, the better. The old procedure sometimes requires months before sufficient skin is available for grafting. In the meantime, some patients may die due to trauma to the body or infections that might set in.

An additional problem with the existing skin culture technique is that the skin that grows is generally not very uniform. In fact, it is both lumpy and full of holes. While this is cosmetically undesirable, it is also potentially dangerous to the patient. The holes provide sites for infection by foreign organisms. In addition, the lumpy skin is more susceptible to tearing from stress and stretching.

One procedure used to grow uniform tissue is to grow the skin cells on top of a matrix of 3T3 cells. Rheinwald and Green (1975). The 3T3 cells act as a feeder layer for the skin cells. The skin cells are able to grow to a confluent mono-layer on top of the 3T3 cells, which become part of the skin tissue.

This procedure cannot be used safely for skin grafts, however. The 3T3 cells are partially transformed cells. This means that they are more susceptible to carcinogenic material than non-transformed cells, and might more readily become cancerous. Since skin is constantly exposed to many carcinogenic factors, such as the ultraviolet light in sunlight, the presence of 3T3 cells in skin grafts could pose a real cancer risk to the patients.

Other potentially dangerous method have been developed in an effort to grow smooth, uniform sheets of epidermis. Cholera toxin has been added to the tissue culture medium in order to promote growth of keratinocytes. Green (1978). Understandably this is dangerous to graft recipients because any toxin remaining in the tissue when it is grafted could prove lethal, or at least damaging, to the recipient.

Currently, a need exists for a method of culturing sheets of skin that are uniform in texture and do not cause rejection by the patient's immune system. This procedure must not depend on cancer promoting agents, such as 3T3 cells, nor on dangerous chemicals, such as cholera toxin. It would also be desirable to be able to store such skin sheets so that they would be available upon demand for grafting onto wounds. This would alleviate much risk to the patients, potentially saving many lives of victims of burns and other severe skin wounds.

Summary of Invention

This invention provides a method for culturing and storing sheets of epidermis to be used in skin grafts. It has several advantages over the previously described methods. In this method cells can be grown to a relatively uniform sheet, without holes or lumps. Thus this method provides a more durable and more protective sheet of skin than previously available.

This method also does not use any subdermal matrix, such as a feeder layer of 3T3 cells. As a result, this method creates a much smaller risk of cancer formation than do other described procedures. This method also does not reply on dangerous chemicals, such as cholera toxin, thereby presenting less of a risk of illness or death to the patient.

This method also overcomes the possibility of rejection of the graft by the patient's immune system. As a result, sheets of epidermis can be grown from tissue from sources other than the graft recipient. This allows for a tremendous increase in starting material, and therefore a tremendous increase in supply of epidermal sheets for grafts.

This method further provides a means for long-term storage of epidermal sheets. As a result, epidermal sheets can be readily available for immediate use on victums needing skin grafts. Such rapid availability will decrease trauma to the victims as well as protect them from infection. It will allow much more rapid healing of skin wounds.

Detailed Description

This invention has seven steps. In Step 1, cells are prepared for primary culture. In Step 2 primary cultures are subdivided (passaged). In step 3 these sub-cultures are grown to confluent monolayers in the presence of EGF (epidermal growth factor). In step 4 the media is shifted, and the monolayer grows to multi-layered epidermis. In step 5 the resulting sheet of epidermis is frozen and stored. In step 6 the frozen tissue is defrosted. In step 7 the cultured epidermal tissue is used on a patient as a skin graft. Steps one through four are modifications of the procedure of Pittelkow and Scott (1986) Mayo Clinic Proceedings 61 771, the disclosure of which is incorporated herein by reference.

In step one, starting material is obtained for growth of the epidermal sheets. Any source of epidermal tissue will work, such as surviving undamaged skin of the patient to receive the graft. In the example, foreskins obtained from healthy adult male humans are used, as they are readily available. It is important to note that the source of the tissue need not be the patient who will receive the graft of cultured epidermal sheet.

The skin tissue is then separated into its two components, epidermis and dermis, by incubating the tissue in a digestive solution. The dermis is discarded. The cells of the epidermis are then separated from each other. This can be done by any conventional procedure for starting primary cultures, such as further digestion of the tissue. In the example, tissue is further digested, and then separated to single cells by shearing in a pipet.

An important element of this invention is a method for preparing the tissue culture containers to be used for keratinocyte growth. As noted before, many workers have tried various techniques to help the keratinocytes attach to the culture dishes and grow, the only partial success. In some cases the sheets were not uniform; in other cases dangerous materials were used.

This invention uses a procedure of coating the culture containers with collagen type I to aid cell adherence and growth. A sterile solution of collagen type I is placed in the culture dish and then vaporized, leaving a coating of collagen type I on the growth surface. The dishes are then resterilized by irradiation before use.

The separated cells are then cultured in MCDB 153 or any other suitable cell culture medium without serum. This allows the cells of the primary culture to divide and multiply in number.

Step two allows further amplifications of cell number. This step is not necessary to the growth of epidermal sheets, but it allows cells from a small piece of tissue to be used for production of a large number of epidermal sheets. In this second step, cells are harvested from the primary culture. This can be done by standard methods. In the example, cells are digested and then pipetted to form single-cell suspensions. These cells are then divided so that the cells from one primary culture dish now can seed many dishes to grow to confluency.

This division procedure is called one cell passage. Different types of primary cells can be passaged different numbers of times without dying. In the example, keratinocytes from human foreskins can be passaged up to four times to increase the starting material for monolayer growth.

In step three of the invention, primary or passaged cells are grown from single-cell suspensions to confluent monolayers on tissue culture dishes. These cells are grown in the presence of epidermal growth factor (EGF) and absence of serum, in any suitable cell culture medium. In the example, complete MCDB 153 medium, containing elevated levels of several amino acids, was used. The media is supplemented with EGF between 5ng/ml and 50ng/ml. In the example 10ng/ml was used. The EGF allows the cells to continue to divide after the point when they would stop dividing in the absence of EGF. Thus, a uniform monolayer of cells grows in the presence of EGF.

In this step, cells are grown to between 80% of confluency and three days after confluency. In the example, the preferable length is until the cells reach 100% confluency. The less confluency the monolayer, the more likely is the resulting tissue to tear. After cells confluency they can be maintained for a few days. After that the cells will start to die, creating weak spots in the monolayer.

Step four is then performed. The medium is switched to culture medium containing serum, with or without EGF. In the example, Dulbecco's modified Eagle medium (DMEM) supplemented with 10% fetal calf serum (FCS) was used. Any appropriate cell culture medium will work. Fetal calf serum concentrated can be as low as 5%; however the cells will grow very slowly then. Above 20% the cost of fetal calf serum becomes an inhibiting factor.

In this step the cell monolayer grows to an epidermal sheet of cells, around six to twelve layers thick. This growth takes around four to five days in medium with 10% FCS. This epidermal sheet is of uniform thickness and is capable of mimicking native skin when used in a graft.

When examined under an electron microscope, the epidermal sheet appeared like normal tissue. The cell structures in the lower three or four layers appeared normal. Cell organelles, such as free ribosomes, tonofibrils, and mitochondria were scattered throughout the cytoplasm. The nuclei of the cells appeared ovoid in shape with smooth contour nuclear membranes. Desmosomes with regular structure between the cell-cell junctions were also present as in the native skin. In the top two layers of cells, normal fibrillar structures were randomly distributed in the cytoplasm.

Cells from epidermal sheets were also stained and viewed under the light microscope. This test showed that the cells had the normal number of chromosomes (23 pairs in humans).

Samples of epidermal sheets grown in this manner were tested for carcinogenicity in nude mice. Nude mice have impaired immune systems and are therefore especially susceptible to carcinogens. Mice that had injections of cells from these epidermal sheets showed no evidence of tumors after five months.

In addition the tissue was examined for cell type at various stages of growth. The cells were stained with fluorescent antibody labels to detect proteins produced specifically by different types of skin cells. The cells were then examined under a fluorescent microscope. This showed that the sheets were around 99% pure, containing the type of cell isolated for the initial growth. In the example the tissue was around 99% keratinocytes.

This cell purity is an important factor in eliminating immune rejection of the tissue in a patient receiving a skin graft. This is preferably due to the fact that keratinocytes do not express the human major histocompatibility complex. As a result, keratinocytes are not recognized by the patient's body as foreign material, and the patient's immune system is not triggered to fight off the new cells.

This invention for the first time recognizes this lack of immune response. As a result, this invention allows the cultured tissue to be used as allografts on patients other than those from whom the original tissue was taken. This invention thus encompasses the culturing of any epidermal cells that do not express the major histocompatibility complex, such as melanocytes.

Step five of the invention provides a way to store cultured epidermal sheets so thay they will be available for immediate use when the need for skin grafts arises. In this step the tissue is released from the culture dish. First the culture medium is washed from the sheets of cells and replaced with digestion solution #3. This solution is approximately 1-10% Dispase in phosphate buffered saline (PBS) or medium. The incubation time will depend on how high the concentration and what the Dispase is dissolved in. Too high a concentration or too long an incubation will start to break apart the intercellular bonds, dissolving the tissue into individual cells. In the example, Dispase is dissolved at 1% concentration in PBS, and the tissue is incubated for one to two hours. This releases the tissue from the culture dish.

After this digestion the digestion solution is washed out and replaced with freezing solution. The purpose of this solution is to protect the tissue from damage that would be caused by ice crystals in the cells. Therefore a solution is used that will not form such crystals. Freezing solutions in use for cell suspensions can be used to freeze the tissue.

The freezing solution can contain dimethyl sulfoxide (DMSO) at a concentration of 3-10%, preferably 5%, and glycerol at a concentration of 5-20%, preferably 20%. These are dissolved in fresh culture medium. The freezing solution is then added to the washed tissue in the culture dish, and the dish is sealed to prevent spilling and/or contamination. One method of sealing is to wrap paraffin film around the dish.

The dish is then subjected to a gradual decrease in temperature from room temperature to -135 C. This can be done in increments, such as by placing the dish in a -20 C freezer for several hours, followed by a -70 C freezer overnight, and ultimately transferred to a -135 C freezer. A better method is to place the dish in a freezer that will start at room temperature and decrease 1 C per minute until -135 C is reached. This gradual decrease in temperature helps to prevent the formation of ice crystals.

Frozen sheets of epidermis can be stored at -135 C for at least two years. When defrosted these sheets are as functional as freshly grown sheets.

Step six of the invention is accomplished by rapidly defrosting the frozen epidermal sheets. This may be done by providing an ambient temperature of at least 25 C. Wrapped culture dishes are transferred from the freezer to a 37 C water bath, in the preferred embodiment of the invention. An incubator, either air or water, between 30 C and 40 C would also work. However, the air incubators do not conduct heat as rapidly as water baths, and incubators below 37 C will also defrost the tissue more slowly. An incubator that is too warm (above 40 C) might overheat the cells.

When the freezing solution inside the culture dish has melted, the tissue sheets have defrosted. These sheets will float on the freezing solution. This solution is then washed out with fresh culture medium, and then with phosphate buffered saline (PBS). The epidermal sheets are now ready for step seven.

In step seven the sheets of epidermis are used for skin grafts within about twenty four hours after they are defrosted. The medium is sterilely removed, such as by suction. Sterile gauze is then placed on the sheet, which will adhere to the gauze. Then the tissue is placed, gauze side up, over the wound. The tissue will automatically graft itself to adjacent skin.

These epidermal sheets can be used for autografts or allografts. Tests in humans have so far shown a complete absence of rejection when used as allografts. This invention for the first time is believed to provide a completely successful solution to the problem of immune rejection of allografts.

## Example

This invention uses the method of Pittelkow and Scott (1986) as modified herein.

Foreskins were obtained from healthy young adult male humans via circumcision. The specimens were thoroughly sterilized and then chopped into small pieces in petri dish under sterile conditions. The pieces of samples were trypsinized with 0.17% trypsin for 10-12 hours at 4°C. The thin, translucent epidermis was separated from its underlying dermis by lifting the epidermis with sterile forceps. The epidermis was further treated with 0.75% trypsin for 1-2 hours at 37°C and pipetted into a single cell suspension. The viable epidermal cells were counted with a hemocytometer.

Tissue culture dishes were coated with collagen type I. One half milliliter of a 0.1% solution was placed in each 75 cm² dish. The solution was dried by evaporating the water for three days in a laminar flow hood. The dishes were then resterilized by irradiation at a factory specializing in this procedure.

Cells were seeded at 5X10⁴ viable cells/cm² in complete MCDB 153 medium. This medium is identical to that used by Pittelkow and Scott except that the porcine pituitary gland extract (140-160mg protein/ml) replaced the bovine pituitary gland extract. The remainder of the culturing procedure is as in Pittelkow and Scott.

After the tissue was released from the culture dish with Dispase, some of it was frozen. The tissue to be frozen was washed with fresh DMEM, and the culture medium was replaced with DMEM containing 5% DMSO and 20% glycerol. The dish was sealed with paraffin film and placed in a special freezer. The tissue was cooled incrementally at a rate of -1°C per minute until it reached -135°C. The tissue was then stored for two years at -135°C.

Culture dishes were removed from the freezer and the tissue rapidly defrosted by placing the sealed dishes in a 37°C water bath. When the medium had melted and the tissue was floating, it was defrosted. The freezing solution was then washed from the tissue with fresh DMEM, and replace with PBS. The tissue was then incubated at 37°C until use, which was less than 24 hours after thawing.

Sterile gauze was applied to the surface of the tissue, and the tissue was transformed directly to a clean debrided wound. The tissue automatically adhered to the wound. Three days later the gauze was removed. The tissue then became part of the healing wound. Any tissue that overlapped native skin dried up and fell off, leaving no scar at the edge of the wound. Twelve months later there were still no signs of rejection.

It is to be understood that the present invention is not limited to the specific embodiments set forth above, but is of the full scope of the appended claims.

**Claims**

1. A method for culturing mammalian epidermal cells suitable for an allograft comprising:

    a. growing the cells on a collagen type I treated surface in the absence of serum, in the presence of a growth medium comprising epidermal growth factor (EGF), to form an approximately confluent layer of cells;

    b. causing the growth medium to be a serum-containing medium; and

    c. growing the approximately confluent cell layer to a plurality of layers.

2. The method of claim 1 where the cells are keratinocytes.

3. The method of claim 1 or 2 where the source of cells is human skin.

4. The method of claim 3 where the source of cells is human foreskins.

5. The method of any one of the preceding claims where the concentration of EGF is between 5 ng/ml and 50 ng/ml, preferably between 10 ng/ml and 40 ng/ml, most preferably 10 ng/ml.

6. The method of any one of the preceding claims where the collagen treatment comprises coating collagen type I on the surface at between 0.1 mg and 10 mg per 75 $cm^2$ of surface preferably at 0.5 mg per 75 $cm^2$.

7. The method of claim 1 where growth medium is modified MCDB 153.

8. The method of claim 7 where the modification comprises porcine pituitary gland extract replacing bovine pituitary gland extract.

9. The method of any one of the preceding claims where cells are grown to between 80% of confluency and 3 days post-confluency, preferably to 100% confluency.

10. The method of any one of the preceding claims where the serum-containing medium is Dulbecco's modified Eagle medium (DMEM).

11. A method for storing a cultured epidermal sheet for a skin graft comprising:

    a. releasing the cultured epidermal sheet from a culture surface;

    b. freezing the epidermal sheet;

12. The method of claim 11 where the cultured epidermal sheet is digested with Dispase to release it from the surface.

13. The method of claim 12 where Dispase is used at 1-10% for 1-2 hours.

14. The method of any one of claims 11 to 13 where the freezing comprises placing the cultured epidermal sheet in freezing solution and then exposing it to incremental decreases in temperature.

15. The method of claim 14 where the freezing solution is tissue culture medium containing 3-10% dimethyl sulfoxide (DMSO), preferably 5% DMSO, and containing 5-20% glycerol, preferably 20% glycerol.

16. The method of claim 14 or 15 where the decrease in temperature comprises placing the cultured epidermal sheet at approximately -20°C for a period of time, preferably 1-2 hours, then at approximately -70°C for a period of time, preferably 6-18 hours, and then transferred to approximately -135°C.

17. The method of claim 14 or 15 where the decrease in temperature comprises placing the cultured epidermal sheet in a freezer that drops in temperature approximately 1°C per minute, to approximately -135°C.

18. A method according to any one of claims 11 to 17 in which the frozen epidermal sheet is stored for a period of time and then rapidly defrosted.

19. The method of claim 18 where the frozen cultured epidermal sheet is stored from 1 hour to at least 2 years.

20. The method of any one of claims 18 to 19 where the frozen cultured epidermal sheet is defrosted at 30°C-40°C, preferably at 37°C.

21. The method of any one of claims 18 to 20 where the frozen cultured epidermal sheet is defrosted in an air or water incubator, preferably a water incubator.

22. The method of any one of claims 11 to 21 in which the cultured epidermal sheet is prepared according to the process of any one of claims 1 to 10.

23. A frozen cultured epidermal sheet.

24. A cultured epidermal sheet produced by a process according to any one of claims 1 to 10 for use as a skin graft.

25. A cultured epidermal sheet according to claim 24 for use as an allograft.

26. A cultured epidermal sheet according to claim 24 or 25 stored according to any one of claims 11 to 21.

27. A cultured epidermal sheet according to any one of claims 24 to 26 on which has been placed gauze.